# FASCICULE DE BREVET EUROPEEN

(11) **EP 3 773 353 B1**
(45) Date de publication et mention de la délivrance du brevet: **09.03.2022**
(21) Numéro de dépôt: 19718783.4
(22) Date de dépôt: 21.03.2019
(51) Int. Cl.: A61F 2/50, A61F 2/78, B29C 70/44

(54) **PROCEDE DE FABRICATION D'UN MANCHON SUR MESURE POUR PROTHESE**
VERFAHREN ZUR HERSTELLUNG EINER ANGEPASSTEN HÜLSE FÜR EINE PROTHESE
METHOD FOR MANUFACTURING A CUSTOMISED SLEEVE FOR A PROSTHESIS

(30) Priorité: 26.03.2018 FR 1852594
(43) Date de publication de la demande: 17.02.2021
(73) Titulaire: Paillet, Stephane, 26230 Valaurie (FR)
(72) Inventeur: PAILLET, Stéphane, 84820 VISAN (FR)
(74) Mandataire: IP Trust
(86) Numéro de dépôt international: PCT/FR2019/050646
(87) Numéro de publication internationale: WO 2019/186033

(56) Documents cités:
- EP-A1- 0 976 371
- FR-A1- 2 994 079
- US-A- 5 728 168
- US-A1- 2003 181 989
- US-A1- 2008 234 836
- US-A1- 2015 079 014

## Description

### DOMAINE DE L'INVENTION

La présente invention concerne un procédé de fabrication d'un manchon sur mesure pour prothèse, ainsi qu'un manchon obtenu par ledit procédé.

### ETAT DE LA TECHNIQUE

Lorsqu'un individu ayant subi une amputation d'une extrémité d'un membre doit être équipé d'une prothèse, il est habituel de placer un manchon entre le moignon et la prothèse. Ledit manchon sert d'interface cutanée entre la prothèse et le membre, visant à améliorer la tenue de la prothèse sur la peau et à améliorer le confort de l'individu.

Le manchon doit être spécialement adapté à la taille et à la forme du membre de l'individu.

Une première possibilité est de sélectionner, parmi une gamme de manchons standards existant dans différentes tailles, celui qui est le plus adapté à l'individu, à partir de mesures prises sur l'individu.

Cependant, il est rare, souvent difficile, de pouvoir trouver parmi ces manchons standards un manchon qui convienne parfaitement à l'individu.

Pour pallier cet inconvénient, il est connu de fabriquer le manchon sur mesure, à partir de dimensions prises sur l'individu ou à partir d'un moulage du membre à appareiller.

Pour minimiser le temps et le coût de confection d'un tel manchon, des procédés consistent à utiliser une préforme fabriquée au préalable, pouvant se présenter en plusieurs tailles standardisées parmi lesquelles on peut choisir la plus adaptée à l'individu, et de façonner ladite préforme afin de l'adapter au membre de l'individu.

Ainsi, par exemple, le document FR 2 799 953 décrit un procédé de fabrication d'un manchon comprenant la fourniture d'une préforme thermoformable, notamment en une mousse de polyoléfine ou en éthylène vinyl acétate (EVA), le chauffage de ladite préforme pour la ramollir, puis l'enfilage de la préforme directement sur le moignon de l'individu ou sur un moule définissant la forme extérieure dudit moignon.

Le document FR 2 994 079 décrit quant à lui un procédé de fabrication d'un manchon comprenant la fourniture d'une préforme thermoformable, notamment en un copolymère styrène-éthylène-butylène-styrène (SEBS), réalisée par injection plastique et présente une épaisseur uniforme, la mise en place de ladite préforme sur un moule définissant une réduction du moignon, le chauffage de l'ensemble préforme/moule et le démoulage après refroidissement.

Cependant, ces procédés ne permettent de contrôler que la forme intérieure du manchon, qui est définie par la surface extérieure du moule. Par ailleurs, ces procédés de formage ne permettent pas d'intégrer aisément des composants au manchon. De tels composants peuvent être par exemple des renforts, un tissu anti-élongation, une cupule agencée au niveau du moignon, etc. L'intégration de ces composants nécessite des opérations de reprise, notamment de collage, sur le manchon après formage.

Une autre technique consiste à fabriquer un contre-moule définissant la forme extérieure du manchon et à injecter du silicone liquide dans l'intervalle située entre ce contre-moule et le moule, qui forme une empreinte de moulage. Après solidification, le manchon en silicone ainsi obtenu est démoulé. Des renforts ou un tissu anti-élongation peuvent éventuellement être insérés dans l'empreinte avant l'injection du silicone liquide ou lors d'une seconde injection de renforcement. Cependant, ce procédé est relativement long puisqu'il nécessite plusieurs opérations préalables pour fabriquer le contre-moule par thermoformage. Par ailleurs, l'obtention d'une épaisseur régulière dépend fortement de la compétence de l'opérateur ; il est en effet possible qu'en raison d'un mauvais positionnement initial du contre-moule vis-à-vis du moule, l'épaisseur soit trop épaisse dans certaines zones du manchon et trop fine dans d'autres. Dans ce cas, il peut être nécessaire de procéder à des réparations manuelles du manchon après démoulage.

Le document EP 0 976 371 A1 décrit : un procédé de fabrication d'un manchon sur mesure pour une prothèse permettant de s'affranchir de l'utilisation d'un moule. Ce procédé comprend les étapes suivantes :
(a) fourniture d'un moule d'une extrémité d'un membre d'un individu destiné à recevoir ladite prothèse;
(b) fourniture d'une préforme en un matériau élastomère, ladite préforme comprenant une extrémité proximale ouverte et une extrémité distale fermée;
(c) mise en place de ladite préforme sur le moule;
(d) mise en place, sur ladite préforme, d'une couche de tissu et enduction d'un polymère réticulable à température ambiante;
(f) mise en place d'une bâche à vide, mise sous vide puis réticulation du polymère.

### EXPOSE DE L'INVENTION

Un but de l'invention est de concevoir un procédé plus répétable, plus rapide et moins onéreux pour former un manchon prothétique sur mesure.

A cet effet, l'invention propose un procédé de fabrication d'un manchon sur mesure pour une prothèse, comprenant les étapes suivantes :
(a) fourniture d'un moule d'une extrémité d'un membre d'un individu destiné à recevoir ladite prothèse ;
(b) fourniture d'une préforme en un matériau élastomère, ladite préforme comprenant une extrémité proximale ouverte et une extrémité distale fermée ;
(c) mise en place de ladite préforme sur le moule ;
(d) mise en place, sur ladite préforme, d'au moins un élément parmi : un renfort en polymère, une couche d'un tissu anti-élongation, une cupule distale et une gaine de drainage de l'air ;
(e) mise en place d'une bâche à vide autour de la préforme ;
(f) mise sous vide et injection d'un polymère réticulable à température ambiante dans ladite bâche à vide, de sorte à former un revêtement d'épaisseur uniforme sur la préforme et chaque élément mis en place sur ladite préforme.

Par « température ambiante » on entend dans le présent texte une température comprise entre 20 et 25°C.

Par « sur mesure » on entend dans le présent texte que le manchon est spécifiquement adapté à la morphologie de l'individu.

Selon un mode de réalisation, la préforme est en gel de silicone.

Selon un mode de réalisation, le matériau injecté autour de la préforme est un silicone bi-composant.

De manière avantageuse, l'ensemble des étapes peut être mis en oeuvre à une température comprise entre 20 et 25°C.

De manière préférée, la préforme présente une épaisseur variable. De manière avantageuse, la préforme présente une épaisseur plus grande à son extrémité distale qu'à son extrémité proximale.

Avantageusement, le procédé comprend, après la réticulation du polymère injecté autour de la préforme, le collage d'un tissu élastique ou l'application d'une peinture autoglissante sur la surface extérieure du manchon.

Un autre objet concerne un manchon obtenu par le procédé qui vient d'être décrit. Ledit manchon comprend :
- une préforme en un matériau élastomère,
- au moins un élément choisi parmi : un renfort polymère, une couche d'un tissu anti-élongation, une cupule distale et un tissu de drainage de l'air,
- un revêtement d'un polymère réticulable à basse température enrobant la préforme et ledit élément, ledit revêtement présentant une épaisseur uniforme.

### BREVE DESCRIPTION DES FIGURES

D'autres caractéristiques et avantages de l'invention ressortiront de la description détaillée qui va suivre, en référence aux dessins annexés sur lesquels :
- la figure 1 est une vue de côté d'un moule d'un membre à équiper de la prothèse ;
- la figure 2 est une vue de la préforme avant sa mise en place sur le moule de la figure 1 ;
- la figure 3 est une vue de la préforme entourée de la bâche à vide,
- les figures 4 et 5 illustrent deux formes d'exécution différentes d'un manchon fini.

Pour favoriser la lisibilité des figures, les différents éléments ne sont pas nécessairement représentés à l'échelle.

### DESCRIPTION DETAILLEE DE MODES DE REALISATION DE L'INVENTION

L'invention permet d'obtenir une meilleure répétabilité de la qualité du manchon, notamment en termes d'épaisseur, grâce à l'utilisation d'une préforme présentant une qualité constante, et à l'injection d'une couche régulière d'un polymère réticulable à basse température autour de la préforme, de manière à intégrer les différents composants du manchon.

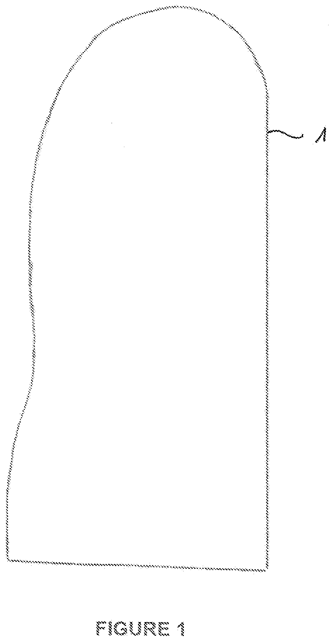

La figure 1 illustre de manière schématique un moule 1 du membre à appareiller.

De manière avantageuse, le moule est réduit par rapport au membre, par application d'un taux de réduction défini par des abaques de rectification qui sont couramment utilisée dans le domaine de la conception de prothèses. L'application d'une telle réduction permet que le manchon final soit monté avec un léger serrage sur le membre, afin d'assurer une bonne tenue du manchon.

Le moule peut être fabriqué par toute technique connue, par exemple à partir d'un moulage du membre, ou à partir d'une image tridimensionnelle dudit membre.

Le moule peut être en résine, en plâtre, en mousse de polyuréthane ou en tout autre matériau adapté à la mise en œuvre du procédé qui va suivre.

On fournit ensuite une préforme réalisée en un matériau élastomère, tel qu'un gel de silicone.

La préforme présente une extrémité proximale ouverte (pour l'enfilage sur le membre) et une extrémité distale fermée (pour loger le moignon). La surface intérieure de la préforme est destinée à être en contact avec la peau pendant le port de la prothèse.

Le polymère constituant la préforme est principalement choisi pour le confort qu'il procure à l'individu, puisqu'il est en contact direct avec la peau. L'homme du métier est en mesure de choisir la dureté appropriée.

Cette préforme est fabriquée au préalable dans une ou plusieurs dimensions standardisées. Elle n'est donc pas spécifique au patient. Cependant, dans le cas où différentes préformes sont disponibles, le praticien peut choisir la plus adaptée en fonction de la morphologie du patient. En général, la préforme présente une circonférence légèrement inférieure à celle du moule, afin d'être montée avec un léger serrage sur celui-ci.

De manière avantageuse, la préforme présente une épaisseur non uniforme avec notamment des zones en surépaisseur pour assurer un meilleur confort du patient. L'épaisseur est ainsi généralement plus importante au niveau de l'extrémité distale de la préforme, et plus fine au niveau de son extrémité proximale.

Selon une variante d'exécution, la préforme présente une épaisseur uniforme.

La préforme est fabriquée de manière industrielle avec des techniques de moulage qui assurent une excellente répétabilité des épaisseurs et, d'une manière plus générale, de la qualité d'une préforme à l'autre.

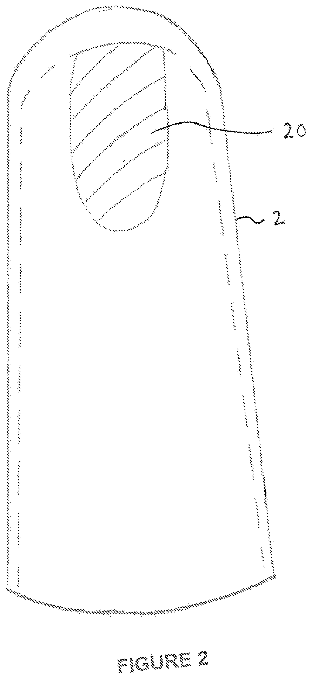

La figure 2 illustre la préforme 2 avant sa mise en place sur le moule 1. Dans l'exemple illustré, la préforme comprend un renfort 20 schématisé par des hachures, constitué par une surépaisseur s'étendant dans une direction longitudinale.

Le praticien positionne alors sur la préforme les différents composants destinés à être intégrés au manchon. Ces composants peuvent comprendre :
- un ou plusieurs renforts, qui se présentent généralement sous la forme de patches de polymère (par exemple de silicone) ;
- une ou plusieurs couches d'un tissu anti-élongation, qui est un tissu étirable dans une seule direction, en vue de permettre de contrôler l'élongation du manchon ;
- une cupule destinée à être positionnée au niveau du moignon et placée par conséquent à l'extrémité distale de la préforme, ladite cupule pouvant être pourvue ou non d'un élément de fixation de la prothèse (par exemple, un embout taraudé destiné à maintenir la prothèse par vissage),
- une gaine, par exemple, en polyamide, présentant des mailles suffisamment larges pour permettre le drainage de l'air contenu entre la préforme et ladite gaine lors de la mise sous vide qui sera effectuée ultérieurement,
- éventuellement, un tissu élastique contribuant à renforcer le manchon et/ou à améliorer son esthétique.

Une fois cet assemblage réalisé, une bâche à vide est mise en place autour de la préforme. De manière connue en elle-même, ladite bâche à vide est en un matériau étanche à l'air et permet de faire le vide autour de la préforme, avant l'injection d'un polymère réticulable à basse température destiné à solidariser l'ensemble des composants à la préforme et donner au manchon sa forme définitive.

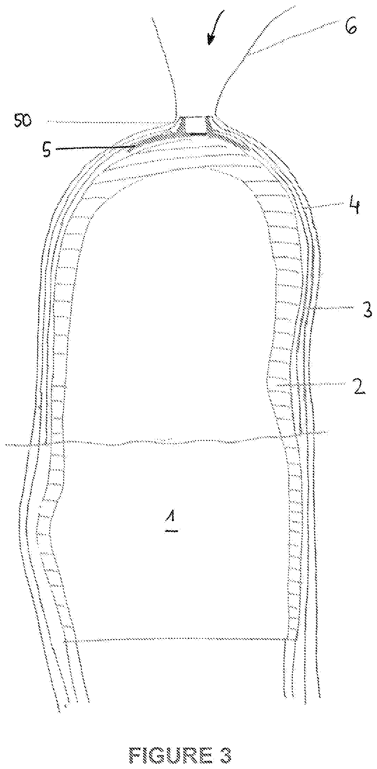

La figure 3 illustre ainsi, à titre d'exemple, la préforme 2 sur le moule 1 avec une couche 3 d'un tissu anti-élongation et une gaine 4 de drainage en polyamide, ainsi qu'une cupule 5 comprenant un embout taraudé 50, entourée d'une bâche à vide 6. L'injection du polymère est schématisée par la flèche.

La dépression appliquée dans la bâche à vide est typiquement de l'ordre de 0 à moins 1013 hPa par rapport à la pression atmosphérique qui est ici considérée égale à 1013 hPa. L'application de cette dépression a pour effet de plaquer les parois de la bâche contre la préforme, la couche de drainage servant à maintenir les composants tout en permettant à l'air interstitiel de s'échapper.

Le polymère injecté dans la bâche à vide est avantageusement du silicone ou un autre polymère réticulable à température ambiante. Un tel silicone est généralement désigné par l'acronyme RTV du terme anglo-saxon « Room Temperature Vulcanizing ». Ce silicone est formé par mélange de deux composants, en présence d'un catalyseur, qui réticulent idéalement à une température comprise entre 20 et 25°C. Ce silicone doit être compatible avec le matériau de la préforme, c'est-à-dire présenter de bonnes qualités d'accroche sur la préforme, afin d'assurer la cohésion du manchon.

L'épaisseur de silicone déposée autour de la préforme est de préférence de l'ordre de 0,1 à 1 mm, mais elle peut être plus épaisse en fonction des éléments placés sur la préforme. La bâche à vide permet d'assurer l'uniformité de la couche de silicone déposée autour de la préforme. Ainsi, la qualité du manchon final ne dépend pas de la compétence de l'opérateur.

La durée nécessaire à la réticulation est de l'ordre de 5 à 60 minutes, mais peut varier fortement en fonction des polymères utilisés.

Ainsi, aucun chauffage n'est nécessaire pour le formage du manchon.

Cependant, il peut être avantageux d'utiliser une étuve pour accélérer le processus de réticulation. De plus, une cuisson des silicones est vivement conseillée.

Une fois le polymère réticulé, l'opérateur retire la bâche à vide et procède le cas échéant à la finition du manchon.

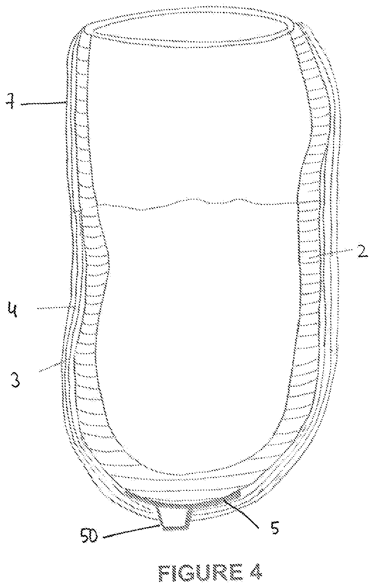

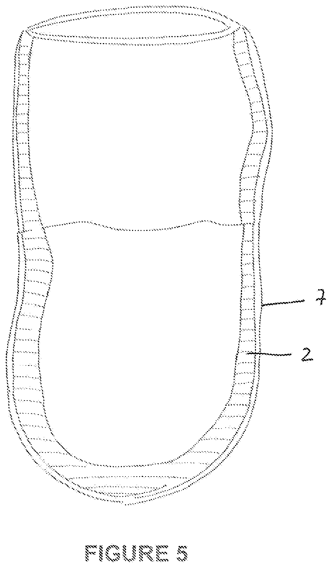

Ladite finition peut consister en le collage d'un tissu à base d'élasthanne (désigné par le repère 7 sur les figures 4 et 5 qui représentent deux versions d'un manchon fini) et/ou en l'application d'une peinture autoglissante. En l'absence d'un tel revêtement favorisant le glissement, on pourra appliquer du talc sur le manchon pour favoriser le glissement du silicone sur lui-même pendant la mise en place du manchon sur le membre à appareiller.

Le procédé selon l'invention permet donc de fabriquer rapidement et à faible coût, un manchon spécifique au porteur de la prothèse et de qualité répétable.

### REFERENCES

FR 2 799 953

FR 2 994 079

## Revendications

1. Procédé de fabrication d'un manchon sur mesure pour une prothèse, comprenant les étapes suivantes :
(a) fourniture d'un moule (1) d'une extrémité d'un membre d'un individu destiné à recevoir ladite prothèse ;
(b) fourniture d'une préforme (2) en un matériau élastomère, ladite préforme comprenant une extrémité proximale ouverte et une extrémité distale fermée ;
(c) mise en place de ladite préforme (2) sur le moule (1) ;
(d) mise en place, sur ladite préforme (2), d'au moins un élément parmi : un renfort en polymère, une couche (3) d'un tissu anti-élongation, une cupule distale (5) et une gaine (4) de drainage de l'air ;
(e) mise en place d'une bâche à vide (6) autour de la préforme ;
(f) mise sous vide et injection d'un polymère réticulable à température ambiante dans ladite bâche à vide (6), de sorte à former un revêtement d'épaisseur uniforme sur la préforme (2) et chaque élément mis en place sur ladite préforme.

2. Procédé selon la revendication 1, dans lequel la préforme (2) est en gel de silicone.

3. Procédé selon l'une des revendications 1 à 2, dans lequel le matériau injecté autour de la préforme (2) est un silicone bi-composant.

4. Procédé selon l'une des revendications 1 à 3, dans lequel l'ensemble des étapes est mis en oeuvre à une température comprise entre 20 et 25°C.

5. Procédé selon l'une des revendications 1 à 4, dans lequel la préforme (2) présente une épaisseur variable.

6. Procédé selon la revendication 5, dans laquelle la préforme présente une épaisseur plus grande à son extrémité distale qu'à son extrémité proximale.

7. Procédé selon l'une des revendications 1 à 6, comprenant, après la réticulation du polymère injecté autour de la préforme, le collage d'un tissu élastique (7) ou l'application d'une peinture auto-glissante sur la surface extérieure du manchon.

## Patentansprüche

1. Verfahren zum Herstellen eines maßgefertigten Überzugs für eine Prothese, umfassend die folgenden Schritte:
(a) Bereitstellen eines Abdrucks (1) eines Endes eines Gliedmaßes einer Person, die die Prothese erhalten soll;
(b) Bereitstellen eines Vorformlings (2) aus einem elastomeren Material, wobei der Vorformling ein offenes proximales Ende und ein geschlossenes distales Ende umfasst;
(c) Anbringen des Vorformlings (2) auf dem Abdruck (1);
(d) Anbringen von mindestens einem der folgenden Elemente auf dem Vorformling (2): einer Polymerverstärkung, einer Schicht (3) aus einem dehnungsfesten Gewebe, einer distalen Pfanne (5) und eines Schlauchs (4) zur Drainage von Luft;
(e) Anbringen einer Vakuumabdeckung (6) um den Vorformling herum;
(f) Vakuumieren und Einspritzen eines sich bei Umgebungstemperatur vernetzenden Polymers in die Vakuumabdeckung (6), so dass auf dem Vorformling (2) und jedem auf dem Vorformling angebrachten Element ein Überzug gleichmäßiger Dicke gebildet wird.

2. Verfahren nach Anspruch 1, wobei der Vorformling (2) aus Silikongel besteht.

3. Verfahren nach einem der Ansprüche 1 bis 2, wobei das um den Vorformling (2) eingespritzte Material ein Zweikomponenten-Silikon ist.

4. Verfahren nach einem der Ansprüche 1 bis 3, wobei alle Schritte bei einer Temperatur zwischen 20 und 25 °C durchgeführt werden.

5. Verfahren nach einem der Ansprüche 1 bis 4, wobei der Vorformling (2) eine variable Dicke aufweist.

6. Verfahren nach Anspruch 5, wobei der Vorformling an seinem distalen Ende eine größere Dicke als an seinem proximalen Ende aufweist.

7. Verfahren nach einem der Ansprüche 1 bis 6, umfassend nach dem Vernetzen des um den Vorformling herum eingespritzten Polymers das Verkleben eines elastischen Gewebes (7) oder das Auftragen einer selbstgleitenden Farbe auf die Außenfläche des Überzugs.

## Claims

1. Method for producing a custom-made liner for a prosthesis, comprising the following steps:
(a) providing a mold (1) of an end of a limb of an individual intended for receiving said prosthesis;
(b) providing a preform (2) of an elastomer material, said preform comprising an open proximal end and a closed distal end;
(c) placing said preform (2) on the mold (1);
(d) placing, on said preform (2), at least one element from among a polymer reinforcement, a layer (3) of an anti-elongation fabric, a distal cup (5) and an air drainage casing (4);
(e) placing a vacuum tank (6) around the preform;
(f) creating a vacuum and injecting a polymer which can be cross-linked at room temperature into said vacuum tank (6) so as to form a coating of uniform thickness on the preform (2) and each element placed on said preform.

2. Method according to claim 1, wherein the preform (2) is made of silicone gel.

3. Method according to either claim 1 or claim 2, wherein the material injected around the preform (2) is a two-component silicone.

4. Method according to any of claims 1 to 3, wherein all of the steps are carried out at a temperature of between 20 and 25°C.

5. Method according to any of claims 1 to 4, wherein the preform (2) has a variable thickness.

6. Method according to claim 5, wherein the preform has a greater thickness at its distal end than at its proximal end.

7. Method according to any of claims 1 to 6, comprising, after cross-linking the polymer injected around the preform, gluing an elastic fabric (7) or applying a self-sliding paint to the outer surface of the liner.
